# EUROPÄISCHE PATENTSCHRIFT

(11) **EP 2 964 607 B1**
(45) Veröffentlichungstag und Bekanntmachung des Hinweises auf die Patenterteilung: **08.08.2018**
(21) Anmeldenummer: 14707766.3
(22) Anmeldetag: 04.03.2014
(51) Int. Cl.: C07C 209/26, C07C 211/29

(54) **VERFAHREN ZUR HERSTELLUNG VON HALOGEN-N,N-DIMETHYLBENZYLAMINEN**
PROCESS FOR THE PREPARATION OF HALOGEN-N, N-DIMETHYLBENZYLAMINES
PROCÉDÉ DE FABRICATION D'HALOGÈNE-N, N-DIMÉTHYLBENZYLAMINE

(30) Priorität: 06.03.2013 EP 13158020; 10.09.2013 EP 13183790
(43) Veröffentlichungstag der Anmeldung: 13.01.2016
(73) Patentinhaber: LANXESS Deutschland GmbH, 50569 Köln (DE)
(72) Erfinder: PETERS, Lars, 51371 Leverkusen (DE); SCHULZE TILLING, Andreas, 42799 Leichlingen (DE); STIRNER, Wolfgang, 51467 Bergisch Gladbach (DE)
(86) Internationale Anmeldenummer: PCT/EP2014/054108
(87) Internationale Veröffentlichungsnummer: WO 2014/135508

(56) Entgegenhaltungen:
- EP-A1- 1 201 642
- EP-A2- 0 355 351
- WO-A2-2009/110985
- BHATTACHARYYA, SUKANTA: "A high throughput synthesis of N,N-dimethyl tertiary amines", SYNTHETIC COMMUNICATIONS ( 2000 ), 30(11), 2001 -2008 CODEN: SYNCAV; ISSN: 0039-7911, 2001, XP002698158,

## Beschreibung

Die Erfindung betrifft ein Verfahren zur Herstellung von Halogen-N,N-dimethylbenzyl-aminen mit Halogen = Chlor (Chlor-N,N-dimethylbenzylamin, Cl-DMBA) durch reduktive Aminierung. Das erfindungsgemäße Verfahren hat dabei den Vorteil, dass es in Abwesenheit von Schwefel durchgeführt werden kann.

Es ist bekannt, dass aus einem Aldehyd und einem sekundären Amin zunächst ein Halbaminal gebildet wird (Aminierung), welches unter Abspaltung von Wasser auch mit einem weiteren Molekül des Amins zum Aminal weiterreagieren kann.

Alle genannten Umsetzungsprodukte (Halbaminal bzw. Aminal) können katalytisch zu den entsprechenden Aminen hydriert werden. Diese Reduktion ist beispielsweise beschrieben in Houben-Weyl, Methoden der Organischen Chemie, 4. Auflage, Band IV/1c (1980), S. 127/128, 239/240 u. 436. Solche katalytischen Hydrierungen sollen auch unter Erhaltung von Halogen möglich sein. Jedoch ist dieses Ergebnis auf die Benutzung von Palladium-Katalysatoren beschränkt, und es wird weiter darauf hingewiesen, dass man vorteilhafterweise mit desaktiviertem Katalysator und bei niederen Temperaturen arbeitet (Houben-Weyl, Methoden der Organischen Chemie, 4. Auflage, Band IV/1c (1980), Seite 240). Auch unter Verwendung anderer Katalysatoren, wie Platin oder Raney-Nickel soll Halogen erhalten bleiben. Die an den zitieren Stellen angegebenen speziellen Hydrierungen stellen jedoch keine systematische Untersuchung dar (Houben-Weyl, Methoden der Organischen Chemie, 4. Auflage, Band IV/1c (1980), Seite 436, Absatz 3) und zeigen zum Teil überaus mäßige Ausbeuten, wie im Falle des p-Chlorbenzyl-methylketons, welches nur in 10% der theoretischen Ausbeute in das zugehörige Amin übergeführt werden kann (Houben-Weyl, Methoden der Organischen Chemie, 4. Auflage, Band IV/1c (1980), Seite 436, unten). Insbesondere bei der Herstellung stark basischer Amine muss mit Nebenprodukten gerechnet werden (Houben-Weyl, Methoden der Organischen Chemie, 4. Auflage, Band IV/1c (1980), Seite 240, Absatz 2).

Aus EP-A-0 355 351 ist die katalytische Hydrierung in Gegenwart von Schwefel in Form organischer Schwefelverbindungen bekannt. Schwefelverbindungen haben jedoch den Nachteil, dass diese genau zudosiert werden müssen, da diese bei einer Fehl-/Überdosierung den Katalysator vergiften. Schwefelverbindungen haben zudem negative Auswirkungen auf das Endprodukt, insbesondere auf Chlor-N,N-dimethylbenzylamin, so dass diese aufwändig abgetrennt werden müssen.

Des Weiteren ist aus WO 2009/ 110985 bekannt, Halogen-N,N-dimethylbenzylamine mit Halogen = Brom aus Natrium-Cyano-Borhydrid herzustellen. Dieses Verfahren hat jedoch die Nachteile, dass Cyanide freigesetzt werden, da Natrium-Cyano-Borhydrid in hohem Überschuss eingesetzt werden muss und dadurch bedingt eine hohe Salzfracht anfällt, die aufwändig entsorgt werden muss. Großtechnisch sind diese Verfahren aufgrund der hohen Salzfracht und der Giftigkeit des Natrium-Cyano-Borhydrids nicht geeignet. Alternativverfahren, wie in EP-A-0355315 beschrieben, sehen den Zusatz von festen Co-Katalysatoren vor, die den Nachteil haben, dass diese ausbluten können, mit der Folge, dass sich diese als unerwünschtes Nebenprodukte im Endprodukt wiederfinden. S. Bhattacharyya, Synthetic Communications, 30(11), 2001-2008 (2000) beschreibt die Herstellung N,N-Dimethylgruppen-enthaltender tertiärer Amine durch reduktive Aminierung von Carbonylverbindungen mit Titanium(IV)isopropoxid und Natriumborhydrid.

Bei den Verfahren gemäß Stand der Technik werden entweder teure Edelmetallkatalysatoren verwendet, nur geringe Ausbeuten erreicht, oder die zugesetzten Additive müssen aufwändig abgetrennt werden, was diese Verfahren häufig unwirtschaftlich macht.

Vor dem Hintergrund des vorstehend genannten Standes der Technik bestand daher die Aufgabe, ein besonders wirtschaftliches Verfahren bereitzustellen, das die Herstellung von Halogen-N,N-dimethylbenzylamine mit Halogen = Cl bevorzugt ortho-Chlor-N,N-dimethylbenzylamin (o-CI-DMBA), in hohen Ausbeuten und in Abwesenheit von Schwefel und schwefelhaltigen Verbindungen oder anderer Katalysatorgifte bzw. Additive, die die Katalysatoraktivität vermindern, erlaubt.

Es wurde überraschend gefunden, dass die Nachteile des Standes der Technik vermieden werden können und Halogen-N,N-dimethylbenzylamine mit Halogen = Cl in hoher Ausbeute und in Abwesenheit von Schwefel hergestellt werden können, wenn bei der reduktiven Aminierung ein bestimmtes molares Verhältnis von Halogen-Benzaldehyd mit Halogen = Cl zu Dimethylamin eingehalten wird.

Gegenstand der vorliegenden Erfindung ist daher ein Verfahren zur Herstellung von Halogen-N,N-dimethylbenzylaminen mit Halogen = Cl vorzugsweise o-, m- oder p-Chlor-N,N-dimethylbenzylamin, ganz besonders bevorzugt o-Chlor-N,N-dimethylbenzylamin, durch reduktive Aminierung von Halogenbenzaldehyd mit Halogen = Chlor mit Dimethylamin in Gegenwart eines Katalysators, ausgewählt aus der Gruppe Palladium, Platin, Ruthenium, Nickel- oder Cobalt oder Ni-haltige und/oder Co-haltigen Katalysatoren, vorzugsweise eines Ni-haltigen und/oder Co-haltigen Katalysators, wobei die Reaktion in Gegenwart von Ameisensäure, Essigsäure oder Propionsäure, bevorzugt Essigsäure, durchgeführt wird, und wobei das molare Verhältnis von Halogen-Benzaldehyd mit Halogen = Cl zu Dimethylamin 1:2-5 beträgt. Halogen kann dabei in o- (= ortho), m-(= meta) oder p-(= para)-Position, vorzugsweise in o-Position sein.

Bei den eingesetzten Halogenbenzaldehyden handelt es sich um handelsübliche Verbindungen, die z.B. unter den Namen 2-Chlorbenzaldehyd bei der Merck Millipore GmbH oder 3-Chlor- bzw. 4-Chlorbenzaldehyd bei der Alfa Aesar GmbH & Co KG kommerziell erhältlich sind.

Bei dem eingesetzten Dimethylamin handelt es sich um eine handelsübliche Verbindung, die unter den Namen Dimethylamin wasserfrei 2.8 (99,8%ig) bei GHC Gerling, Holz und Co. Handels GmbH, kommerziell erhältlich ist.

Als Katalysatoren werden erfindungsgemäß Palladium, Platin, Ruthenium sowie Nickel und/oder Kobalt eingesetzt. Bevorzugt sind Ni- und/oder Co-Katalysatoren, erzeugt durch Auslaugen von Ni- oder Co-Legierungen, Ni oder Co auf Trägern, in Form von Skelettkatalysatoren, elementarem Ni(Co)-Schwamm, als Ni-Oxid, Co-Oxid, Raney-Nickel, Raney-Kobalt. Träger sind beispielsweise SiO₂, Al₂O₃, Bims, Kohle und andere dem Fachmann bekannte Träger. Besonders bevorzugt sind dabei Katalysatoren, wie Ni- oder Co-Katalysatoren, erzeugt durch Auslaugen von Ni oder Co-Legierungen, die als Bestandteil der Legierung unter anderem Legierung von Nickel, Kobalt, Nickel-Eisen, Nickel-Kobalt oder Nickel-Eisen-Kobalt in wasserfreier oder auch wasserfeuchter oder lösungsmittelfeuchter Form eingesetzt. Die Ni- und Co-haltigen Katalysatoren können auch gemeinsam eingesetzt werden.

In einer bevorzugten Ausführungsform der Erfindung wird das Verfahren in Abwesenheit von Cokatalysatoren, ausgewählt aus der Gruppe der Metalloxide oder Metallmischoxide, Zeolithen, Metall- oder Ammoniumsalzen von Mineralsäuren oder organischen Säuren, sauren Ionenaustauschern oder Gemischen davon, durchgeführt.

Unter Skelettkatalysator werden solche Katalysatoren verstanden, die durch Auslaugen mit starken Basen, vorzugsweise mit konzentrierter Natronlauge entstanden sind. Die dadurch entstehende poröse Struktur wird auch Skelettstruktur genannt.

Der Katalysator wird dabei vorzugsweise in einer Menge von 0,1-25 Gew.-%, bevorzugt 1,5-12,5 Gew.-%, bezogen auf das zu hydrierende Substrat, eingesetzt.

In besonders bevorzugter Weise werden Ni-haltige Skelett-Katalysatoren eingesetzt. Bei den Katalysatoren handelt es sich um handelsübliche Katalysatoren, wie sie z.B. bei der Firma H.C. Starck GmbH hergestellt werden.

Die Reaktion kann dabei in Anwesenheit oder aber auch in Abwesenheit von Lösungsmitteln erfolgen, wobei die Abwesenheit von Lösungsmitteln bevorzugt ist.

Als Lösungsmitteln können Alkohole, vorzugsweise Methanol, Ethanol, Isopropanol, Butanol, aliphatische oder aromatische Kohlenwasserstoffe, vorzugsweise Toluol, Xylol, Cyclohexan, Isooctan und ähnliche, Ether, wie Tetrahydrofuran, Dioxan oder Methyl-tert.-butylester, Ester wie Ethylacetat und schließlich das Reaktionsprodukt selbst, sofern es bei der Reaktionstemperatur flüssig ist, eingesetzt werden. Ein Gehalt an Wasser (z.B. bis zu 20 Gew.-%) stört nicht, insbesondere, wenn das Reaktionsmedium mit Wasser mischbar ist.

Die Hydrierung wird vorzugsweise bei 50-150°C, und einem H₂-Druck von 10-220 bar, durchgeführt.

Bei dem für die Hydrierung eingesetzten Wasserstoff handelt es sich vorzugsweise um kommerziell erhältlichen Wasserstoff.

Im Allgemeinen wird das erfindungsgemäße Verfahren so durchgeführt, dass man Dimethylamin und den Halogenbenzaldehyd, die Säure, gegebenenfalls das Lösungsmittel und den Katalysator, in einem Hydrierautoklaven vorlegt und nach Verschließen des Reaktors die Luft mit Inertgas, vorzugsweise Argon und Stickstoff, versetzt, und anschließend das Inertgas mit Wasserstoff verdrängt. Mit Wasserstoff wird anschließend der gewünschte Druck eingestellt. In einer bevorzugten Ausführungsform der Erfindung wird solange nachgerührt bis der Druck des nachgeführten Wasserstoffs konstant bleibt. Nach beendeter Reaktion (Wasserstoffaufnahme) wird das Reaktionsgefäß zunächst entspannt, vorzugsweise auf Raumtemperatur abgekühlt und entleert; der Katalysator wird abfiltriert und kann ohne Aufbereitung wiederverwendet werden.

Das Verfahren kann sowohl diskontinuierlich als auch kontinuierlich, vorzugsweise in einem Festbettreaktor, durchgeführt werden.

In einer bevorzugten Ausführungsform wird bei der reduktiven Aminierung als Säure Essigsäure zugesetzt. Dabei handelt es sich um handelsübliche Essigsäure, in einem Konzentrationsbereich von 0,1 bis 10%, die z.B. bei der Firma Merck Millipore erhältlich ist. In einer bevorzugten Ausführungsform der Erfindung beträgt der Gehalt an Essigsäure vorzugsweise 0,5 - 5 Gew.%, besonders bevorzugt 0,5 - 4 Gew.%.

In einer weiteren bevorzugten Ausführungsform der Erfindung wird das Verfahren in Abwesenheit von Schwefel durchgeführt.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Die nachfolgenden Beispiele dienen der Erläuterung der Erfindung, ohne dabei limitierend zu wirken.

### Beispiele:

In einem 0,7 L VA Autoklav wurden 150 g (1,06 mol) o-Chlorbenzaldehyd, 3 g (0,05 mol) Eisessig und 6 g Nickelkatalysator (ein Katalysator der Firma HC Starck GmbH) zusammen vorgelegt. Der Autoklav wurde geschlossen, bei Raumtemperatur wurden 213,6 g (4,74 mol) Dimethylamin aufgedrückt. Mit Wasserstoff wurde anschließend ein Druck von 50 bar eingestellt und auf 100°C aufgeheizt. Nach Erreichen der Zieltemperatur steigerte man den Innendruck mit Wasserstoff auf 200 bar und hielt diesen. Sobald der Druck des nachgeführten Wasserstoffs konstant blieb, wurde 30 min bei 100°C nachgerührt. Nach beendeter Wasserstoffaufnahme und Abkühlen auf Raumtemperatur wurde die Reaktionsmischung ausgenommen (294,5 g Rohprodukt, ohne Aufarbeitung) und feste Anteile an Katalysator und Nebenprodukten per Filtration abgetrennt. Das Produkt wird gaschromatographisch analysiert und darauf basierend die Ausbeute bestimmt.

| **Versuch** | **Lösungsmittel** | **E/V** | **Parameter** | **molares Verhältnis o-Cl-Benzaldehyd : Dimethylamin** | **Ausbeute o-Cl-N,N-dimethyl-benzylamin** |
|---|---|---|---|---|---|
| 1 | ohne | V | 50bar, 150°C, | 1 : 1,5 | 0,7 |
| 2 | ohne | E | 50bar, 150°C | 1 : 4,5 | 55,5 |
| 3 | ohne | V | 150bar, 150°C | 1 : 1,5 | 50,7 |
| 4 | ohne | E | 150bar, 150°C | 1 : 4,5 | 73,3 |
| 5 | ohne | V | 50bar, 90°C | 1 : 1,5 | 78,1 |
| 6 | ohne | E | 50bar, 90°C | 1 : 4,5 | 81,9 |
| 7 | ohne | V | 150bar, 90°C | 1 : 1,5 | 81,1 |
| 8 | ohne | E | 150bar, 90°C | 1 : 4,5 | 90,6 |
| 9 | ohne | E | 100bar, 120°C | 1 : 3 | 85,5 |

| | | | | | |
|---|---|---|---|---|---|
| V = Vergleich, E = erfindungsgemäß | | | | | |

Aus den Beispielen ist klar ersichtlich, dass bei einem molaren Verhältnis von o-Chlorbenzaldehyd zu Dimethylamin von 1 : 2-5 deutlich bessere Ausbeuten erzielt werden.

## Patentansprüche

1. Verfahren zur Herstellung von Halogen-N,N-dimethylbenzylaminen mit Halogen = Chlor durch reduktive Aminierung von Halogenbenzaldehyd mit Halogen = Chlor mit Dimethylamin in Gegenwart eines Katalysators, ausgewählt aus der Gruppe Palladium, Platin, Ruthenium, Nickel- oder Cobalt oder Ni-haltige und/oder Co-haltigen Katalysatoren, **dadurch gekennzeichnet, dass** die Reaktion in Gegenwart von Säuren ausgewählt aus der Gruppe Ameisensäure, Essigsäure und Propionsäure bei einem H₂-Druck von 10 bis 220 bar bei einer Temperatur von 50 bis 150°C durchgeführt wird, wobei das Halogenbenzaldehyd mit Dimethylamin in einem molaren Verhältnis von 1:2 - 5, umgesetzt wird.

2. Verfahren nach Anspruch 1, **dadurch gekennzeichnet, dass** dieses in Abwesenheit von Cokatalysatoren, ausgewählt aus der Gruppe der Metalloxide oder Metallmischoxide, Zeolithen, Metall- oder Ammoniumsalzen von Mineralsäuren oder organischen Säuren, sauren Ionenaustauschern oder Gemischen davon, durchgeführt wird.

3. Verfahren nach einem der Ansprüche 1 bis 2, **dadurch gekennzeichnet, dass** als Katalysatoren Ni- oder Co-Katalysatoren, erzeugt durch Auslaugen von Ni oder Co-Legierungen, Ni oder Co auf Trägern ausgewählt aus der Gruppe SiO₂, Al₂O₃, Bims, Kohle in Form von Skelettkatalysatoren, elementarem Ni(Co)-Schwamm, als Ni-Oxid, Co-oxid, Raney-Nickel, Raney-Kobalt verwendet werden.

4. Verfahren nach einem der Ansprüche 1 bis 3, **dadurch gekennzeichnet, dass** der Katalysator in Mengen von 0,1-25 Gew.-%, bevorzugt 1,5-12,5 Gew.-%, bezogen auf das zu hydrierende Substrat, eingesetzt wird.

5. Verfahren nach einem der Ansprüche 1 bis 4, **dadurch gekennzeichnet, dass** die Säure, vorzugsweise Essigsäure, im Bereich von 0,1 - 10 Gew.%, bevorzugt 0,1 - 5 Gew.%, besonders bevorzugt 0,5 - 4 Gew.%, eingesetzt wird.

6. Verfahren nach einem der Ansprüche 1 bis 5, **dadurch gekennzeichnet, dass** dieses ohne Lösungsmittel durchgeführt wird.

7. Verfahren nach einem der Ansprüche 1 bis 5, **dadurch gekennzeichnet, dass** dieses in Anwesenheit mindestens eins Lösungsmittels, ausgewählt aus der Gruppe Methanol, Ethanol, Isopropanol, Butanol, Toluol, Xylol, Cyclohexan, Isooctan, Tetrahydrofuran, Dioxan oder Methyl-tert.-butylester oder Ethylacetat, durchgeführt wird.

8. Verfahren nach einem der Ansprüche 1 bis 7, **dadurch gekennzeichnet, dass** dieses in Abwesenheit von Schwefel durchgeführt wird.

## Claims

1. Method for producing halogen-N,N-dimethylbenzylamines wherein halogen = chlorine by reductive amination of halogenbenzaldehyde wherein halogen = chlorine with dimethylamine in the presence of a catalyst selected from the group palladium, platinum, ruthenium, nickel or cobalt or Ni-containing and/or Co-containing catalysts, **characterized in that** the reaction is carried out in the presence of acids selected from the group of formic acid, acetic acid and propionic acid at an H₂ pressure of 10 to 220 bar at a temperature of 50 to 150°C, wherein the halogenbenzaldehyde is reacted with dimethylamine in a molar ratio of 1:2-5.

2. Method according to Claim 1, **characterized in that** it is carried out in the absence of cocatalysts selected from the group of metal oxides or metal mixed oxides, zeolites, metal or ammonium salts of mineral acids or organic acids, acidic ion exchangers or mixtures thereof.

3. Method according to one of Claims 1 to 2, **characterized in that** the catalysts used are Ni or Co catalysts produced by leaching out from Ni or Co alloys, Ni or Co on supports selected from the group SiO₂, Al₂O₃, pumice, carbon in the form of skeletal catalysts, elemental Ni(Co) sponge, as Ni oxide, Co oxide, Raney nickel, Raney cobalt.

4. Method according to one of Claims 1 to 3, **characterized in that** the catalyst is used in amounts of 0.1-25% by weight, preferably 1.5-12.5% by weight, based on the substrate to be hydrogenated.

5. Method according to one of Claims 1 to 4, **characterized in that** the acid, preferably acetic acid, is used in the range from 0.1-10% by weight, preferably 0.1-5% by weight, particularly preferably 0.5-4% by weight.

6. Method according to one of Claims 1 to 5, **characterized in that** it is carried out without solvents.

7. Method according to one of Claims 1 to 5, **characterized in that** it is carried out in the presence of at least one solvent selected from the group methanol, ethanol, isopropanol, butanol, toluene, xylene, cyclohexane, isooctane, tetrahydrofuran, dioxane or methyl tert-butyl ester or ethyl acetate.

8. Method according to one of Claims 1 to 7, **characterized in that** it is carried out in the absence of sulfur.

## Revendications

1. Procédé de fabrication d'halogéno-N,N-diméthylbenzylamines avec halogène = chlore par amination réductrice d'halogénobenzaldéhyde avec halogène = chlore avec de la diméthylamine en présence d'un catalyseur choisi dans le groupe constitué par le palladium, le platine, le ruthénium, le nickel ou le cobalt ou les catalyseurs contenant Ni et/ou Co, **caractérisé en ce que** la réaction est réalisée en présence d'acides choisis dans le groupe constitué par l'acide formique, l'acide acétique et l'acide propionique à une pression d'H₂ de 10 à 220 bar à une température de 50 à 150 °C, l'halogénobenzaldéhyde étant mis en réaction avec la diméthylamine en un rapport molaire de 1:2 à 5.

2. Procédé selon la revendication 1, **caractérisé en ce que** celui-ci est réalisé en l'absence de co-catalyseurs choisis dans le groupe constitué par les oxydes de métaux ou les oxydes mixtes de métaux, les zéolithes, les sels de métaux ou d'ammonium d'acides minéraux ou d'acides organiques, les échangeurs d'ions acides ou leurs mélanges.

3. Procédé selon l'une quelconque des revendications 1 à 2, **caractérisé en ce que** des catalyseurs de Ni ou Co, formés par lixiviation d'alliages de Ni ou Co, Ni ou Co sur des supports choisis dans le groupe constitué par SiO₂, Al₂O₃, la pierre ponce, le charbon sous la forme de catalyseurs à squelette, une éponge de Ni(Co) élémentaire, sous la forme d'un oxyde de Ni, oxyde de Co, nickel de Raney, cobalt de Raney, sont utilisés en tant que catalyseurs.

4. Procédé selon l'une quelconque des revendications 1 à 3, **caractérisé en ce que** le catalyseur est utilisé en quantités de 0,1 à 25 % en poids, de préférence de 1,5 à 12,5 % en poids, par rapport au substrat à hydrogéner.

5. Procédé selon l'une quelconque des revendications 1 à 4, **caractérisé en ce que** l'acide, de préférence l'acide acétique, est utilisé dans la plage allant de 0,1 à 10 % en poids, de préférence de 0,1 à 5 % en poids, de manière particulièrement préférée de 0,5 à 4 % en poids.

6. Procédé selon l'une quelconque des revendications 1 à 5, **caractérisé en ce que** celui-ci est réalisé sans solvant.

7. Procédé selon l'une quelconque des revendications 1 à 5, **caractérisé en ce que** celui-ci est réalisé en présence d'au moins un solvant, choisi dans le groupe constitué par le méthanol, l'éthanol, l'isopropanol, le butanol, le toluène, le xylène, le cyclohexane, l'isooctane, le tétrahydrofurane, le dioxane ou l'ester de méthyle et de tert.-butyle ou l'acétate d'éthyle.

8. Procédé selon l'une quelconque des revendications 1 à 7, **caractérisé en ce que** celui-ci est réalisé en l'absence de soufre.
